# FASCICULE DE BREVET EUROPEEN

(11) **EP 0 575 454 B1**
(45) Date de publication et mention de la délivrance du brevet: **27.08.1997**
(21) Numéro de dépôt: 92907164.5
(22) Date de dépôt: 24.02.1992
(51) Int. Cl.: C07D 207/16, A61K 7/48, A61K 7/06, A61K 7/00, A61K 9/127, A61K 31/40

(54) **UTILISATION DE LA CUCURBITINE POUR LA PREPARATION D'UNE COMPOSITION COSMETIQUE OU PHARMACEUTIQUE, NOTAMMENT DERMATOLOGIQUE, ANTI-ALLERGIQUE, ET PROCEDE EN COMPORTANT APPLICATION**
VERWENDUNG VON CUCURBITINE ZUR HERSTELLUNG EINER KOSMETISCHEN ODER PHARMAZEUTISCHEN ZUSAMMENSETZUNG ZUR DERMATOLOGISCHEN, ANTI-ALLERGISCHEN ANWENDUNG
USE OF CUCURBITINE FOR THE PREPARATION OF A COSMETIC OR PHARMACEUTICAL, PARTICULARLY DERMATOLOGICAL, ANTIALLERGIC COMPOSITION AND METHOD INVOLVING APPLICATION THEREOF

(30) Priorité: 28.02.1991 FR 9102420
(43) Date de publication de la demande: 29.12.1993
(73) Titulaire: PARFUMS CHRISTIAN DIOR, 75008 Paris (FR)
(72) Inventeur: RENIMEL, Isabelle, F-45470 Trainou (FR); ANDRE, Patrice, F-45170 Neuvilles-aux-Bois (FR); THIERY, Valérie, F-45370 Clery-St.-André (FR); GUILLAUMET, Gérald, F-45100 Orléans (FR)
(74) Mandataire: Portal, Gérard
(86) Numéro de dépôt international: FR9200164
(87) Numéro de publication internationale: WO9215563

(56) Documents cités:
- FR-A- 2 522 500
- DATABASE WPI Week 47, Derwent Publications Ltd., London, GB; AN 87-329987 & JP,A,62 234 013 (OSAKA YAKUHIN KENKY) 14 Janvier 1987
- CHEMICAL ABSTRACTS, vol. 82, no. 23, 9 Juin 1975, Columbus, Ohio, US; abstract no. 149446, GONZALEZ ET. AL. 'Pharmacological (anthelminthic) study of Cucurbita maxima seeds and their active principle, cucurbitin.' & AN. R. ACAD. FARM. vol. 40, no. 3-4 pages 475 - 486
- CHEMICAL ABSTRACTS, vol. 112, no. 10, 5 Mars 1990, Columbus, Ohio, US; abstract no. 84295, NESTEROVA ET. AL. 'Development of standardisation methods for kukurbin from pumkin seeds.' & FARMATSIYA vol. 38, no. 6 , MOSCOW pages 36 - 8
- SCIENTIA SINICA vol. X , 1961 pages 852 - 859 T. SUN ET. AL. 'Chemical studies on cucurbita moschata duch,'
- JOURNAL OF THE CHEMICAL SOCIETY, CHEMICAL COMMUNICATIONS. 1973 , LETCHWORTH GB page 2 H. J. MONTEIRO 'New Synthesis of the amino acid (+-)-Cucurbitine.'
- CHEMICAL AND PHARMACEUTICAL BULLETIN. vol. 35, no. 9 , Septembre 1987 , TOKYO JP pages 3845 - 3849 Y. MORIMOTO 'Enzymes and catalysts. II.'
- J. FALBE 'Methoden der Organischen Chemie, Band E5' , GEORG THIEME VERLAG , STUTTGART voir page 534 - page 543
- Week 22, 1977 Derwent Publications Ltd., London, GB; AN 77-39162Y & JP,A,52 051 033 (OGAWA) 23 Avril 1977
- 1987 Derwent Publications Ltd., London, GB; AN 87-273386 & JP,A,62 087 241 (LION CORP.) 21 Avril 1987
- 1987 Derwent Publications Ltd., London, GB; AN 87-105806 & JP,A,62 053 911 (SHISEIDO) 9 Mars 1987
- 1987 Derwent Publications Ltd., London, GB; AN 87-032891 & JP,A,61 289 021 (SHISEIDO) 19 Décembre 1986
- CHEMICAL ABSTRACTS, vol. 112, no. 10, 5 Mars 1990, Columbus, Ohio, US; abstract no. 84295, NESTEROVA ET. AL. 'Development of standardisation methods for kukurbin from pumkin seeds.' & FARMATSIYA vol. 38, no. 6 , MOSCOW pages 36 - 8

## Description

La présente invention concerne essentiellement l'utilisation de la cucurbitine pour la préparation de compositions cosmétiques ou pharmaceutiques, notamment dermatologiques, antiallergiques, et un procédé en comportant application.

La cucurbitine, ou acide 3-amine-3-pyrrolidinecarboxylique, de formule I, suivante : 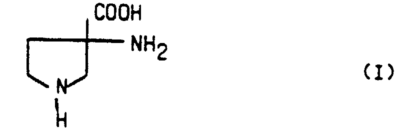 est un acide aminé naturel soluble dans l'eau que l'on trouve dans les cucurbitacées (voir V.H. Mihranian et al., LLOYDIA (1968), 31. (1) 23-29).

La cucurbitine est connue comme anti-parasitaire, en particulier comme anthelmintique contre Schistosoma japonicum. (Morimoto Y. et al., Chem. Pharm. Bull. (1987) 35 (9) 3845-3849).

La cucurbitine peut être obtenue par voie d'extraction sous forme lévogyre ou par voie de synthèse sous forme racémique. Parmi les diverses méthodes de synthèse de cucurbitine, on peut citer particulièment la méthode de synthèse de H.J. Monteiro, J. Chem. Soc., Chem. Commun. (1973) 2. Cette méthode ne conduit qu'à des rendements relativement faibles en cucurbitine racémique. Une autre méthode de synthèse permet d'obtenir séparément l'un et l'autre isomère optique de la cucurbitine. Il s'agit de la méthode de Morimoto et al., Chem. Pharm. Bull. (1987) 35 (9) 3845-3849, qui est une méthode de synthèse par voie enzymatique stéréospécifique par l'emploi d'une estérase de foie de porc. Cette méthode est cependant compliquée, et nécessite un nombre d'étapes relativement élevé.

Il est connu, par les documents FR-A-2 522 500 et JP-A-622 34 013, l'utilisation d'un extrait de Cucurbitacée en général pour la préparation d'une composition cosmétique ou pharmaceutique à activité antiallergique.

Il a maintenant été découvert de manière inattendue que la cucurbitine inhibait la formation d'histamine, médiateur bien connu des allergies, et présentait donc une activité hypohistaminémiante précieuse. Cette activité hypohistaminémiante résulte de l'action inhibitrice de la cucurbitine sur l'histidine décarboxylase qui est l'enzyme responsable de la transformation de l'histidine en histamine. De ce fait, l'administration de la cucurbitine contribue à diminuer la concentration d'histamine dans le sérum sanguin et les tissus.

Ceci constitue un progrès technique important car les manifestations allergiques, notamment les allergies pulmonaires et cutanées, posent aujourd'hui de nombreux problèmes aux thérapeutes qui disposent d'un nombre limité de substances actives et en outre, certaines de ces substances peuvent présenter des effets secondaires. Ainsi, la mise au point d'une nouvelle composition préventive et curative des allergies constitue donc un besoin important.

Ainsi, la présente invention a pour objet principal de résoudre le problème technique consistant en la fourniture d'une solution permettant d'inhiber la formation d'histamine, agent médiateur dans le cadre des manifestations allergiques, afin de permettre la prévention et le traitement des manifestations allergiques.

Selon un autre aspect, la présente invention a pour objet principal de résoudre le problème technique consistant en la fourniture d'une solution permettant de prévenir et de traiter les manifestations allergiques.

La présente invention a encore pour but de résoudre le nouveau prolème technique consistant en la fourniture d'uune solution permettant de diminuer le potentiel allergisant des compositions cosmétiques ou pharmaceutiques, notamment dermatologiques.

La présente invention a encore pour but de résoudre le nouveau problème technique consistant en la fourniture d'une solution permettant de réaliser la synthèse de la cucurbitine par un procédé de synthèse simple, nécessitant un minimum d'étapes, en de bons rendements.

La présente invention permet de résoudre l'ensemble de ces problèmes techniques d'une manière simple, fiable et reproductible, utilisahble à l'échelle industrielle,

Ainsi, selon un premier aspect, la présente invention concerne l'utilisation de la cucurbitine ou de l'un de ses sels ou esters, ou d'un extrait de plante en contenant, à condition qu'un extrait de pulpe de fruits de Cucurbitacée contienne au moins 0,5 % en poids en cucurbitine, comme agent cosmétique.

L'invention concerne aussi l'utilisation de la cucurbitine ou l'un de ses sels ou esters, ou d'un extrait de plante en contenant, à la condition qu'un extrait de pulpe de fruits de Cucurbitacée contienne au moins 0,5 % en poids en cucurbitine, pour la préparation d'une composition pharmaceutique, notamment dermatologique, à activité antiallergique.

Selon une variante de réalisation particulière, l'utilisation est sous forme d'une composition, et dans ce cas, il s'agit de la préparation d'une composition cosmétique ou pharmaceutique, notamment dermatologique, destinée à la prévention ou au traitement symptomatique des manifestations allergiques, quelle qu'en soit l'origine et le point d'application, noamment les bronches, la peau et l'oeil. Ainsi, ladite composition est destinée notamment à la prévention ou au traitement symptomatique de l'asthme bronchique allergique ou de l'effort, du rhume des foins, des rhinites et trachéites spasmodiques, de l'urticaire autres éruptions allergiques, de l'eczéma, rougeurs ou irritations cutanées d'origine allergique, des prurits, de l'oedème de Quincke, des conjonctivites allergiques, ainsi que des réactions allergiques d'origine médicamenteuse.

Dans le domaine plus particulier de la cosmétologie, ladite composition est destinée avantageusement aux lignes de prooduits hypoallergéniques ou pour peaux sensibles ou irritables.

La cucurbitine peut être utilisée soit sous forme libre, soit sous forme de l'un de ses sels ou de ses esters cosmétiquement ou pharmaceutiquement, notamment dermatologiquement, acceptables. Les sels et esters précités peuvent être préparés par des procédés classiques bien connus de l'homme de l'art. Parmi les sels, citons le mono- et dibromohydrate et le mono- et dichlorhydrate. Parmi les esters, citons l'ester méthylique et l'ester éthylique.

Suivant une variante avantageuse, l'extrait de plante précité contenant de la cucurbitine est un extrait de cucurbitacée, en particulier de Cucurbita maxima Duch., de Cucurbita pepo L. ou de Cucurbita moschata Duch ; de préférence, il s'agit d'un extrait de pépins ou de pulpe de fruits. Encore de préférence, il s'agit d'un extrait de pépins de Cucurbitacée.

Selon une variante particulière, l'extrait de plante précité est un extrait de pulpe de fruits de Cucurbitacée contenant au moins 0,5 % en poids en cucurbitine.

Selon une variante de réalisation avantageuse, la cucurbitine ou l'un de sels ou de ses esters cosmétiquement ou pharmaceutiquement, notamment dermatologiquement acceptable, est présente à une concentration de 0,001 % à 10 %, de préférence de 0,01 % à 5 %, en poids de la composition totale.

Selon un deuxième aspect, la présente invention concerne aussi une composition cosmétique, caractérisée en ce qu'elle comprend, comme ingrédient actif, de la cucurbitine ou l'un de ses sels ou esters cosmétiquement acceptable ou un extrait de plante en contenant, à la condition qu'un extrait de pulpe de fruits de Cucurbitacée contienne au moins 0,5 % en poids en cucurbitine, éventuellement dans un excipient, véhicule ou support cosmétiquement acceptable.

Selon une variante avantageuse, l'extrait de plante précité est un extrait de cucurbitacée tel que précédemment défini.

Selon une variante de réalisation préférée, la cucurbitine ou l'un de sels ou esters est présente en une quantité efficace pour présenter une activité antiallergique, en particulier à une concentration de 0,001 *%* à 10 %, de préférence de 0,1 % à 5 % en poids de la composition totale.

Selon un troisième aspect, la présente invention concerne une composition pharmaceutique, notamment dermatologique, de préférence à activité antiallergique, caractérisée en ce qu'elle contient de la cucurbitine ou l'un de ses sels ou esters pharmaceutiquement acceptables, ou un extrait de plante en contenant, à condition qu'un extrait de pulpe de fruits de Cucurbitacée contienne au moins 0,5 % en poids en cucurbitine, en une quantié efficace pour présenter une activité antiallergique, en particulier pour prévenir ou traiter les manifestations allergiques, notamment au niveau des bronches, de la peau et de l'oeil, éventuellement dans un excipient, véhicule ou support pharmaceutiquement acceptable.

Suivant une variante, les compositions cosmétiques et pharmaceutiques précitées contiennent un extrait d'origine végétale tel que précédemment défini.

Selon un quatrième aspect, la présente invention concerne encore un procédé pour diminuer le potentiel allergisant d'une composition cosmétique ou pharmaceutique, notamment dermatologique, caractérisé en ce qu'on incorpore à ladite composition une quantité efficace de cucurbitine sous forme libre ou sous forme de l'un de sels ou esters cosmétiquement ou pharmaceutiquement acceptables, ou d'un extrait végétal en contenant tel que précédemment défini, de sorte que la composition finale présente un risque allergisant réduit.

Dans le cadre de l'un quelconque des aspects précédents, la concentration pondérale préférée en cucurbitine, ou en ses sels ou esters pour les compositions à usage topique, est comprise entre 0,001 % et 10 %, de préférence encore entre 0,01 % et 5 %.

Pour les compositions destinées à l'administration par voie générale (telle que voie orale, parentérale, rectale, inhalations,...) la concentration en cucurbitine n'est pas critique et peut atteindre par exemple 60 % de la compositon. La posologie chez l'homme sera généralement comprise entre 0,1 mg/kg/jour et 20 mg/kg/jour et de préférence entre 1 mg/kg/jour et 15 mg/kg/jour.

Par ailleurs, on utilise généralement la cucurbitine naturelle, de forme lévogyre, ou ses sels ou esters. Des sources de cucurbitine lévogyre particulièrement intéressantes sont la pulpe et les pépins des cucurbitacées, en particulier des espèces Cucurbita pepo L., Cucurbita maxima Duch., Cucurbita moschata Duch. Cependant, on peut également utiliser la forme racémique de la cucurbitine ou de ses sels ou esters.

Par ailleurs, la cucurbitine peut être utilisée sous forme pure, ou sous forme d'extraits, selon l'une quelconque des procédures d'extraction connues l'homme de l'art. Des procédures d'extraction particulièrement intéressantes sont décrites dans la publication de Valentine H. MIHRANIAN et al. dans lloydia 1968, 31, (1) 23-29 qui est incorporée ici par référence, en particulier page 24. Ce procédé prévoit avantageusement de traiter des graines de cucurbitacée décortiquées et dégraissées avec de l'eau avantageusement chauffée au moins 50°C sous agitation constante pendant plusieurs heures. Ensuite le mélange est centrifugé, le surnageant recueilli et les résidus réextraits une ou plusieurs fois de manière similaire avec plusieurs fractions d'eau chauffée. Le surnageant et les lavages sont recombinés puis traités par addition dans un volume équivalent d'éthanol à 85 % pour précipiter les matériaux protéiniques en suspension et l'ensemble est maintenu au réfrigérateur toute la nuit. Le mélange est ensuite centrifugé et le surnageant est recueilli. L'alcool peut être enlevé par distillation par exemple dans un évaporateur rotatif. La solution aqueuse peut être utilisée telle quelle ou bien être ensuite passée sur une colonne de chromatographie par exemple type Dowex 50W-X-8 par exemple mesurant 75 x 2,2 cm. La colonne est lavée avec environ 200 ml d'eau puis éluée avec une solution d'hydroxyde d'ammonium aqueuse à 1 % jusqu'à ce que l'effluent donne une réponse négative au test à la ninhydrine. L'éluat peut être évaporé à sec sous pression réduite éventuellement en chauffant, Le résidu sirupeux peut encore être utilisé tel quel ou bien à nouveau traité à l'eau chaude et avantageusement ajouté en un volume au moins équivalent d'éthanol à 95 %. Ensuite, on peut acidifier goutte à goutte avec un acide pour amener le pH de la solution à pH environ égale à 5, par exemple avec l'acide perchlorique à 60 %. On peut ensuite placer dans un réfrigérateur pendant plusieurs jours pour obtenir un précipité de perchlorate de cucurbitine. Ce précipité peut ensuite être dissous dans quelques millilitres d'eau et éventuellement passé dans une colonne de chromatographie, de préférence de type Amberlite CG-45, par exemple emsurant 20 x 1,5 cm. L'évaporation de l'éluat sous pression réduite donne la cucurbitine à l'état sensiblement pur.

Ainsi, le procédé précédent permet d'obtenir soit de la cucurbitine à l'état pur, soit des extraits ayant des teneurs variables en cucurbitine. Dans le cas où l'on utilise la cucurbitine sous forme d'extait de cucurbitacée, la teneur en cucurbitine est de préférence au moins égale à 0,5 % en poids de l'extrait. Une source préférée d'obtention de cucurbitine est constituée par les pépins de l'espèce Cucurbita, telles que Cucurbita pepo L., Cucurbita maxima Duch. et Cucurbita moschata Duch.

Selon un cinquième aspect, la présente invention permet la mise en oeuvre d'un procédé de traitement d'un être humain ou d'un animal, pour prévenir ou traiter les manifestations allergiques, caractérisé en ce qu'on administre audit être humain ou audit animal une quantité efficace pour prévenir ou traiter les manifestations allergiques de cucurbitine ou de l'un de ses sels ou esters pharmaceutiquement acceptables, ou d'un extrait végétal en contenant tel que précédemment défini.

En particulier, le traitement précité s'applique à la prévention ou au traitement symptomatique de l'asthme bronchique allergique ou de l'effort, du rhume des foins, des rhinites et trachéites spasmodiques, de l'urticaire et autres éruptions allergiques, de l'eczéma, rougeurs ou irritations cutanées d'origine allergique, des prurits, de l'oedème de Quincke, des conjonctivites allergiques, ainsi que des réactions allergiques d'origine médicamenteuse.

Selon une variante de réalisation, on administre la cucurbitine ou l'un de ses sels ou esters pharmaceutiquement acceptables par voie topique à une concentration comprise de préférence entre 0,001% et 10% en poids.

Selon une autre variante de réalisation, on administre la cucurbitine ou l'un de ses sels ou esters pharmaceutiquement acceptables par voie générale à une posologie chez l'homme comprise entre 0,1 mg/kg/jour et 20 mg/kg/jour et de préférence entre 1 mg/kg/jour et 15 mg/kg/jour.

L'invention concerne encore un procédé de préparation d'une composition cosmétique ou pharmaceutique, notamment dermatologique, caractérisé en ce qu'on incorpore la cucurbitine ou l'un de ses sels ou esters cosmétiquement ou pharmaceutiquement acceptables, ou d'un extrait végétal en contenant tel que précédemment défini, dans un support, véhicule ou excipient cosmétiquement ou pharmaceutiquement acceptable.

Selon une variante de réalisation, la cucurbitine ou un de ses sels ou esters cosmétiquement ou pharmaceutiquement acceptables est incorporé dans une formulation cosmétique ou pharmaceutique complète pour diminuer le risque allergisant de celle-ci.

Selon une autre variante de réalisation, on prépare une composition à activité antiallergique. Des variantes de préparation résultent également de la description précédente.

Suivant un mode particulier de réalisation de l'invention dans le cadre de l'un quelconque des aspects exposés plus haut, la composition précitée contenant la cucurbitine ou un de ses sels ou esters ou l'extrait de plante précité contient en outre des vésicules de type liposome. Selon une variante particulière, la cucurbitine, son sel ou ester, est encapsulée, au moins en partie, dans des vésicules de type liposome. Par l'expression "vésicule de type liposome", on entend aussi bien des phases lamellaires lipidiques hydratées que des vésicules lipidiques formées de lipides amphiphiles ioniques ou non-ioniques. Egalement, par l'expression "incorporer au moins en partie dans des vésicules de type liposome", on entend dans la présente description et les revendications que la cucurbitine, son sel ou ester est combiné à des vésicules de type liposome quelle que soit la forme de cette combinaison. Cependant, une combinaison préférée réside dans l'encapsulation de la cucurbitine, son sel ou ester dans des vésicules de type liposome. Mais il n'est pas nécessaire que La totalité soit incorporée ou encapsulée pour obtenir l'effet antiallergique recherché selon l'invention.

On sait que les vésicules de type "liposome" sont préparées à partir de substances lipidiques. Le terme "lipidique" couvre toutes les substances comprenant une chaîne carbonée dite grasse, comportant généralement plus de 5 atomes de carbone, cette substance étant habituellement dénommée "lipide".

Selon l'invention, on utilise titre de lipide, pour former soit les phases lamellaires lipidiques, soit les vésicules de type liposome, des lipides amphiphiles, c'est-à-dire constitués de molécules possédant un groupe hydrophile indifféremment ionique ou non ionique et un groupe lipophile, ces lipides amphiphiles étant susceptibles de former des phases lamellaires lipidiques ou des vésicules de type liposome en présence d'une phase aqueuse.

En particulier, parmi ces lipides, on peut citer : les phospholipides, les phosphoaminolipides, les glycolipides, les alcools gras polyoxyéthylènés, les esters de polyol éventuellement polyoxyéthylénés. De telles substances sont par exemple constituées par une lécitine d'oeuf ou de soja éventuellement hydrogénées, une phosphatidylcholine, une phosphatidylsérine, une sphyngomyéline, un cérébroside ou un stéarate de polyglycérol oxyéthyléné.

L'incorporation dans des phases lamellaires lipidiques hydratées ou dans des liposomes des composés utilisés conformément à la présente invention peut être réalisée selon des techniques de préparation connues, décrites par exemple dans le document US-A-4 508 703 et éventuellement en combinaison avec le document US-A-4 621 023.

Selon un septième aspect, la présente invention couvre encore un procédé de synthèse de la cucurbitine, caractérisé en ce qu'on utilise comme produit de départ de la 1-benzyl-3-pyrrolidinone.

Selon une variante de réalisation particulière de ce procédé de synthèse, on traite la 1-benzyl-3-pyrrolidinone avec une solution ammoniacale de chlorure d'ammonium et de cyanure de potassium, pour obtenir la (±)3-amino-1-benzyl-3-cyano-pyrrolidine. On transforme ensuite ce composé par hydrolyse acide ou basique en acide (±)3-amino-1-benzyl-3-pyrrolidinecarboxylique, et enfin on procède à une réduction hydrogénée, de préférence une hydrogénolyse catalytique pour obtenir l'acide (±)3-amino-3-pyrrolidinecarboxylique ou (±)cucurbitine.

Suivant une variante préférée, la solution ammoniacale précitée est une solution hydroalcoolique, l'alcool étant avantageusement de l'isopropanol ou du méthanol.

Suivant une autre variante préférée, l'hydrolyse précitée est effectuée au moyen d'une solution aqueuse 6 N d'acide bromhydrique.

Selon encore une autre variante, l'hydrogénolyse catalytique est effectuée dans l'eau sous hydrogène pression atmosphérique en présence d'un catalyseur tel que du palladium sur charbon dispersé dans le milieu réactionnel aqueux.

Selon un mode de réalisation préféré du procédé de synthèse, on laisse réagir la 1-benzyl-3-pyrrolidinone avec le chlorure d'ammonium et le cyanure de potassium dans un rapport molaire 1/4/4 à la température ambiante pendant au moins 48 h.

Selon une autre variante de réalisation particulière, on sépare les isomères optiques du mélange racémique, selon toute technique de séparation connue de l'homme de l'art, et en particulier par l'intermédiaire de la préparation de diastéréoisomères.

Le procédé de synthèse de la cucurbitine selon l'invention conduit à des rendements particulièrement élevés en cucurbitine racémique, en moyenne 2 à 3 fois plus élevés que ceux des procédés connus.

D'autres buts, caractéristiques et avantages de la présente invention apparaîtront clairement à la lumière de la description explicative qui va suivre faite en référence à divers exemples de préparation de cucurbitine, ainsi qu'à divers exemples rapportant les résultats d'essais pharmacologiques, ainsi que divers exemples de formulation cosmétique ou pharmaceutique. Dans les exemples, les pourcentages sont donnés en poids, sauf indication contraire.

### Exemple 1

### Synthèse de la cucurbitine sous forme de mélange racémique On procède de la manière suivante :

### a) Synthèse de la (±)3-amino-1-benzyl-3-cyano-pyrrolidine

0,5 g de 1-benzyl-3-pyrrolidinone (2,85 mmol) dissous dans 3 ml de propanol-2 sont ajoutés à une solution de 0,741 g (11,4 mmol) de cyanure de potassium et de 0,615 g (11,4 mmol) de chlorure d'ammonium dans 7 ml d'ammoniaque à 28 %. Le mélange reste à température ambiante sous agitation pendant 3 jours. La solution est lavée avec 15 ml d'une solution de carbonate de potassium à 10 %, et extraite au dichlorométhane (3 xc 15 ml). Après séchage sur sulfate de magnésium et évaporation des solvants, on obtient une huile (0,475 g).

On purifie sur colonne de silice avec dépôt solide. On élue avec un mélange éther-éther de pétrole 4 : 2.

On obtient 0,402 g d'un solide beige (rendement : 70%) constitué par la 3-amino-1-benzyl-3-cyano-pyrrolidine ayant Le spectre RMN suivant :
RNN¹H, 300 MHz, CDCl₃
1,8 (s large, 2H, NH₂); 1,97 (ddd, 1H, J_{4,4'} = 13,4, J_{4,5} = 8, J_{4,5'}=5,4) ; 2,5 (ddd, 1H, J_{4,4'} =8, J_{4,5'}= 8, J_{4',5'} = 5,4) ; 2,64 (d, 1H, J_{2,2'} = 9,4) ; 3,04 (d, 1H, J_{2',2} = 9,4) ; 3,67 (s, 2H, CH₂-C₆H₅); 7,33 (m, 5H, protons aromatiques).

### b) Synthèse de l'acide (±)3-amino-1-benzyl-3-pyrrolidinecarboxylique ou (±)1-benzyl-cucurbitine

On peut réaliser l'hydrolyse du composé précédemment obtenu soit en milieu acide, soit en milieu basique.

### Hydrolyse en milieu acide :

0,3 g (1,49 mmol) de 3-amino-1-benzyl-3-cyano-pyrrolidine, obtenue à l'étape a) dissous dans 5 ml d'acide bromhydrique à 48 %, sont portés à 40-50°C pendant 4 h. Après évaporation de l'acide, on purifie sur colonne de silice. Les impuretés sont éliminées par CH₂Cl₂/MeOH 10 % et L'aminoacide est sorti par MeOH/H₂O 15 %.

Après élimination du méthanol, on décolore le composé au noir animal dans un minimum aqueux à chaud puis on lyophilise.

On obtient un solide jaune foncé (rendement : 80%) constitué par l'acide (±)3-amino-1-benzyl-3-pyrrolidinecarboxylique sous forme mono- ou dibromhydrate ayant le spectre RMN suivant :
RNN¹H, 300 MHz, D₂O
2,47-2,69 (m, 1H, H₄) ; 2,75-2,91 (m, 1H, H₄' ; 3,69-3,94 (m, 3H, H₅ , H_{5'} , H₂) ; 4,12 (d, J_{2,2'} = 14,2, 1H, H_{2'}) ; 4,58 (2d, J = 13,8, 2H, CH₂-C₆H₅) ; 7,62 (s, 5H, protonsaromatiques).

### Hydrolyse en milieu basique :

On dissout 280 mg (1,39 mmol) de 3-amino-1-benzyl-3-cyano-pyrrolidine de l'étape a) dans 2 ml d'éthanol. On ajoute 5 ml d'une solution de soude à 10 % puis on porte à reflux pendant 5 h. Après refroidissement, on acidifie avec de l'acide bromhydrique à 48 %.

On purifie sur colonne de silice : MeOH/H₂O 10 %. On décolore au noir animal puis on lyophilise. La présence des sels de bromure de sodium entraîne l'obtention d'un rendement supérieur à 100 %.

Pour éliminer les sels présents, on prélève 200 mg de pyrrolidine que l'on porte à pH = 8 avec une solution de soude à 10 %. On charge la Duolite Cl⁻ avec une solution d'acide bromhydrique 2N. On élimine les sels avec de l'eau distillée. Pour décrocher la pyrrolidine, on utilise une solution d'acide bromhydrique 0,1 N.

Après lyophilisation, on obtient 120 mg d'acide (±)3-amino-1-benzyl-3-pyrrolidinecarboxylique (rendement : 40%).

### c) Synthèse de l'acide (±)3-amino-3-pyrrolidinecarboxylique ou (±)cucurbitine

300 mg de bromhydrate d'acide (±)3-amino-1-benzyl-3-pyrrolidinecarboxylique obtenu à l'étape b) sont dissous dans 10 ml d'eau. On disperse 0,5 mg de charbon à 10 % de palladium, puis on place cette suspension en atmosphère d'hydrogène sous pression atmosphérique. L'agitation est maintenue pendant 18 h. Après filtration sur papier-filtre et lyophilisation, on obtient un solide jaune (98 %) constitué par l'acide (±)3-amino-3-pyrrolidinecarboxylique ou (±)cucurbitine ayant le spectre RMN suivant :
RMN¹H, 300 MHz, D₂O
2,45 (m, 1H, H₄) ; 2,69 (m, 1H, H_{4'}) ; 3,61 (d, 1H, J_{2,2'} = 13,4) ; 3,67-3,78 (m, 2H, H₅, H_{5'}) ; 4 (d, 1H, J_{2',2} =13,4).

### Exemple 2

### Optimisation du rendement de la synthèse de la (+)cucurbitine

On opère comme indiqué comme à l'exemple 1, en choisissant à l'étape b) l'hydrolyse par l'acide bromhydrique 6 N, en faisant toutefois varier la proportion des réactifs de l'étape a).

Les rendements obtenus figurent au tableau I ci-après.

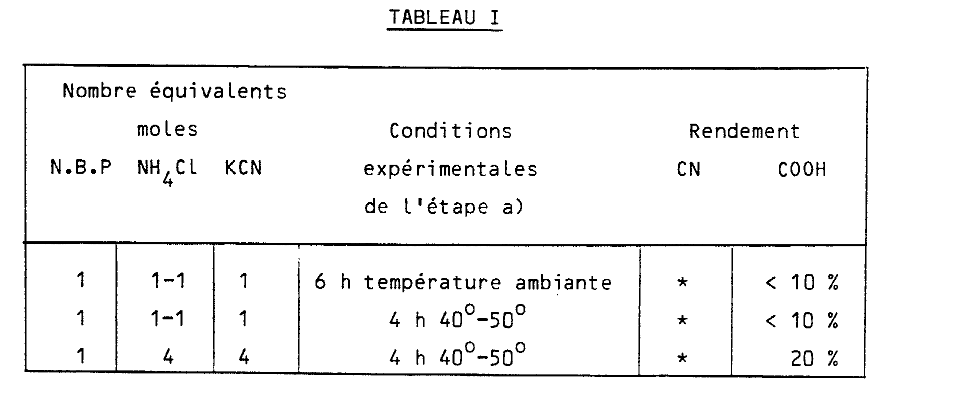

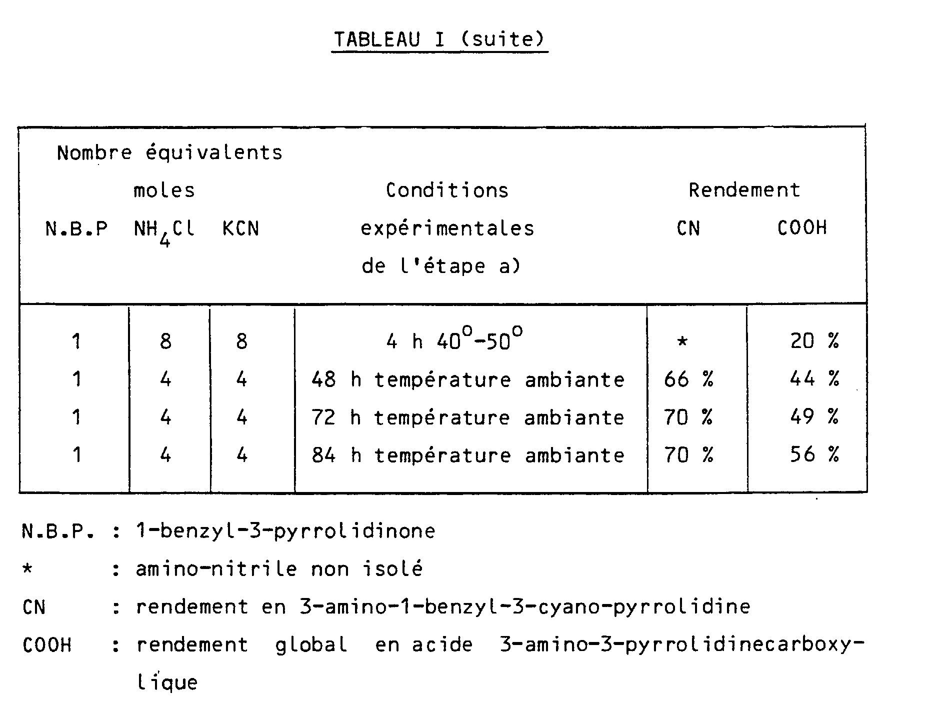

La dernière colonne du tableau I contient les rendements en (±)cucurbitine par rapport aux réactifs de départ.

On observe que les rendements sont maximums si la réaction est menée température ambiante pendant une durée supérieure à 48 h en utilisant des proportions 1/4/4 entre les réactifs : 1-benzyl-3-pyrrolidone, chlorure d'ammonium et cyanure de potassium.

On a par ailleurs observé que l'hydrolyse au moyen de l'acide bromhydrique conduisait également à de meilleurs rendements que lorque l'on utilise de l'acide chlorhydrique, à la même concentration, comme agent d'hydrolyse.

### Exemple 3

### Séparation des isomères optiques de la cucurbitine

La méthode de séparation, connue en elle-même, est basée sur la préparation de dérivés diastéroisomères par couplage de la (±)cucurbitine avec certains réactifs optiquement actifs, après protection de la fonction acide par estérification et de la fonction amine cyclique. Chaque forme de ces stéréoisomères, correspondant à l'un ou l'autre isomère de La cucurbitine, sera isolée par chromatographie, notamment par chromatographie liquide à haute performance ou sur colonne de silice. Pour régénérer ensuite les deux énantiomères de la cucurbitine, il suffit de saponifier l'ester de protection puis d'hydrolyser pour libérer la fonction acide, et de libérer la fonction amine, par exemple par hydrogénolyse comme décrit à l'exemple 1 dans le cas où la fonction amine était protégée par substitution avec un radical benzyle.

Comme réactifs pour la présente méthode, on peut utiliser des composés optiquement actifs lévogyres S, tels que :
- le chlorure de camphanyle-1S,
- l'acide (S)--(-)--α-méthoxy-α-trifluorométhyl-phénylacétique,
- la N-(tertio-butoxycarbonyl)-L-phénylalanine.

Le présent exemple décrit la résolution du racémique de la cucurbitine au moyen du couplage avec le chlorure de camphanyle-1S.

### a) Protection de la fonction acide de la (+)1-benzyl-cucurbitine : synthèse de son ester méthylique

0,1 g de (±)1-benzyl-cucurbitine (0,20 mmol), obtenu à L'étape b de l'exemple 1, est ajouté lentement à une solution glacée de chlorure de thionyle (0,28 mmol) et de méthanol (2 ml). La température de la réaction ne doit pas s'élever au-dessus de -5°C. Le mélange est mis sous agitation à 0°C pendant 2 h, puis ramené à température ambiante pendant deux jours.

Après évaporation, on purifie sur colonne de silice avec dépôt solide. (Eluant CH₂Cl₂/NeOH de 10 à 50 %).

On obtient un produit jaune avec un rendement à 70 %, constitué par l'ester méthylique de la (±)1-benzyl-cucurbitine.

### b) Couplage avec le chlorure de camphanyle-1S (-)

On neutralise 1 mol d'ester méthylique de la (±)1-benzyl-cucurbitine en solution dans 1,5 ml de chlorure de méthylène avec 1 mole de triéthylamine. On ajoute 1,1 équivalent de 1S (-) chlorure de camphanyle. On laisse sous agitation à température ambiante pendant 16 h.

On purifie sur colonne de silice avec dépôt solide (éluant : CH₂Cl₂/MeOH 10 %).

La séparation des diastéréoisomères a été effectuée par H.P.L.C. sur diverses colonnes notamment des Zorbax® NH₂ greffées.

### Exemple 4

### Obtention de cucurbitine à partir de pulpe de Cucurbita pepo

On coupe en deux des fruits frais de Cucurbita pepo, on enlève les pépins qui peuvent servir à la fabrication des extraits de pépins. On broie la pulpe ainsi obtenue que l'on lyophilise. On récupère la poudre que l'on dégraisse à l'éther de pétrole à raison de 1 l pour 100 g de poudre. On récupère l'insoluble par filtration qui constitue l'extrait de pulpe de Curcurbita pepo recherché. On dose la proportion de cucurbitine dans cet extrait par H.P.L.C. et l'on obtient une concentration de 0,03% en poids de cucurbitine dans l'extrait sec dégraissé.

L'extrait sec dégraissé est ensuite introduit dans de l'eau chauffée à environ 50°C sous agitation jusqu'à dissolution complète. On ajoute un volume équivalent d'éthanol à 95 % pour précipiter les matériaux protéiniques en suspension que l'on élimine par centrifugation. Le surnageant clair restant est ensuite acidifié jusqu'à pH 5.0 par de l'acide perchlorique à 60 %. La solution ainsi acidifiée est mise au réfrigérateur pendant au moins 2 jours pour précipiter le perchlorate de cucurbitine que l'on recueille. La cucurbitine peut être obtenue partir de ce perchlorate de cucurbitine de manière classique pour l'homme de l'art, en particulier par passage sur une colonne à résine échangeuse de cations (type Na⁺) et par évaporation de l'éluat sous pression réduite.

### Exemple 5

### Préparation d'un extrait de graines de Cucurbita pepo

On broie 1,5 kg de graines préalablement décortiquées de Cucurbita pepo. La poudre obtenue est soumises trois fois à une extraction à l'hexane (3 litres, 2 litres et 1,8 litre), pour éliminer les matières grasses. Le gâteau séché obtenu est extrait par une solution aqueuse d'acide chlorhydrique maintenue à environ pH 4. Cette extraction est faite en trois fois : deux à température ambiante - environ 22°C -, et la troisième à 70°C. A chaque fois, le maintien en contact du gâteau-solution avec la solution chlorhydrique (2 litres la première fois et 2,5 litres les deux fois suivantes) est de 24 h.

Après essorage, on élimine le résidu solide et on recueille la phase aqueuse que l'on évapore partiellement et que l'on centrifuge. Les culots de centrifugation sont lavés à l'eau distillée, puis éliminés. Les eaux de lavage sont réunies aux concentrats de centrifugation.

La fraction aqueuse est reconcentrée, puis traitée poids pour poids avec de l'éthanol. Il se forme un précipité blanc que l'on élimine par centrifugation. Le surnageant hydro-alcoolique, que l'on neutralise par exemple avec de la soude, constitue un extrait contenant de la (-)-cucurbitine, que l'on peut utiliser en l'état.

On peut aussi évaporer l'alcool, puis atomiser la solution aqueuse obtenue, de manière à obtenir une poudre titrant 3 à 5 % en (-)-cucurbitine, selon les lots de graines utilisés.

### Exemple 6

### Préparation d'un extrait de graines de Cucurbita pepo riche en (-)-cucurbitine, et obtention de (-)-cucurbitine purifiée.

### A - Préparation d'un extrait de graines de cucurbita pepo

La matière première est constituée soit de graines de Cucurbita, décortiquées ou non, ayant une teneur en (-)-cucurbitine de 2 à 4 pour mille en poids, selon le lot ou l'origine, soit de tourteaux de graines de Cucurbita exempts d'huile, à teneur en (-)-cucurbitine de 4 à 8 pour mille en poids, selon le lot ou l'origine.

La matière première est de préférence broyée jusqu'à environ 100 micromètres. Le broyat est ensuite traité par trempage à température ordinaire par de l'eau acidulée (acide sulfurique à 1 pour mille en poids) à pH 3,5 environ, pendant 2 h s'il s'agit de tourteaux broyés ou jusqu'à 16 h s'il s'agit de graines. La quantité d'eau acidulée utilisée est d'environ cinq fois le poids de la matière première. Le tout est ensuite porté à ébullition, à pression atmosphérique, pendant 1 h. Après refroidissement à 80°C, on filtre, puis, éventuellement, on centrifuge. La phase aqueuse obtenue est ensuite portée à une température de 80°C à 90°C, puis microfiltrée sur filtre de 0,5 micromètres. On obtient ainsi un jus titrant environ 10 g de matière sèche par litre.

Ce jus subit ensuite une préconcentration thermique sous vide partiel, jusqu'à une concentration d'environ 250. g/l de matière sèche. Ce préconcentrat est ensuite placé à 4°C pendant 48 h, puis subit une nouvelle concentration sous pression réduite, jusqu'à 500 g de matière sèche par litre, et laissé nouveau au repos à 4°C pendant 48 h. Ces opérations sont suivies d'une filtration sur filtre-presse. On obtient ainsi un extrait contenant de 40 à 50 % de matière sèche et titrant entre 1 et 2 % en (-)-cucurbitine. Les rendements sont d'environ 200 l d'extrait par tonne de matière première.

### B - Obtiention de (-)-cucurbitine purifiée

Le préconcentrat précité, titrant de 250 g à 300 g de matière sèche par litre est neutralisé par de la soude pour obtenir un pH de 7,5. On filtre sur filtre-presse, puis le filtrat est passé sur résine échangeuse de cations (de type Na⁺). L'élution est effectuée au moyen d'une solution d'ammoniaque. Après concentration thermique de l'éluat, sous vide partiel, on obtient un sirop, dont la matière sèche contient environ 50 % en poids de (-)-cucurbitine. Si on le souhaite, ce sirop peut être lyophilisé. Pour cela, on le mélangera avantageusement avec un support poudreux neutre, tel que du talc.

### Exemple 7

### Mise en évidence de l'activité inhibitrice de la formation d'histamine par la cucurbitine

### 1 - A par un test enzymatique

Ce test est basé sur l'action inhibitrice de la cucurbitine sur l'enzyme histidine décarboxylase (HDC) qui transforme l'histidine en histamine, par rapport à celle de la tritoqualine, qui est un inhibiteur connu de l'HDC (voir Carpi C., Maggi G.C. Bull. Soc Ital. Sper. 1968, 44 (6 543-4), et qui est utilisée en thérapeutique comme hypohistaminémiant sous le non de Hypostamine®.

L'activité inhibitrice sur l'HDC peut être aisément réalisée par un dosage colorimétrique sur la base de la réaction chimique suivante :

### Dosage colorimétrique

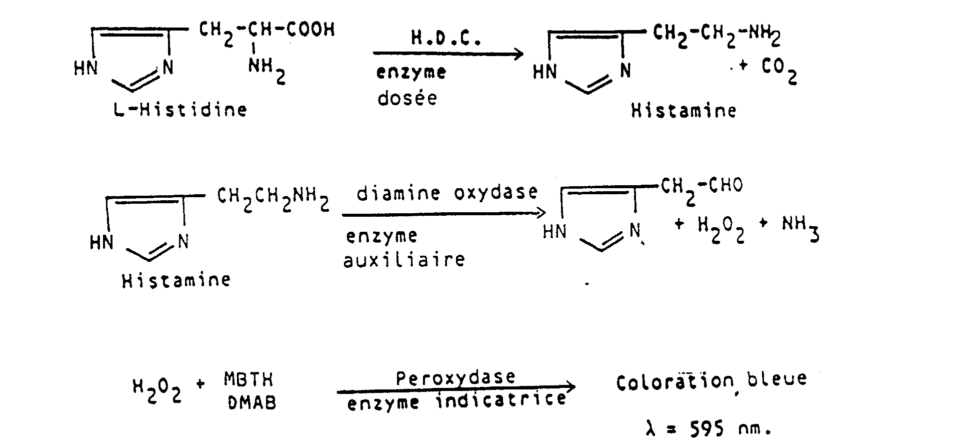

MBTH = 3-méthyl-2-benzothiazolinone hydrazone
DMAB = 3-(diméthylamino)benzoic acid

En pratique, on observe que la formation de la coloration bleue est proportionnelle à la concentration en histidine consommée. On a ainsi pu définir des vitesses initiales de réaction et établir des courbes dites de Mickaelis comme décrit dans Fundamentals of Enzymology, 2nd Ed. Oxford Univ. Press, 1989.

La vitesse initiale est exprimée en variation d'absorbance par minute.

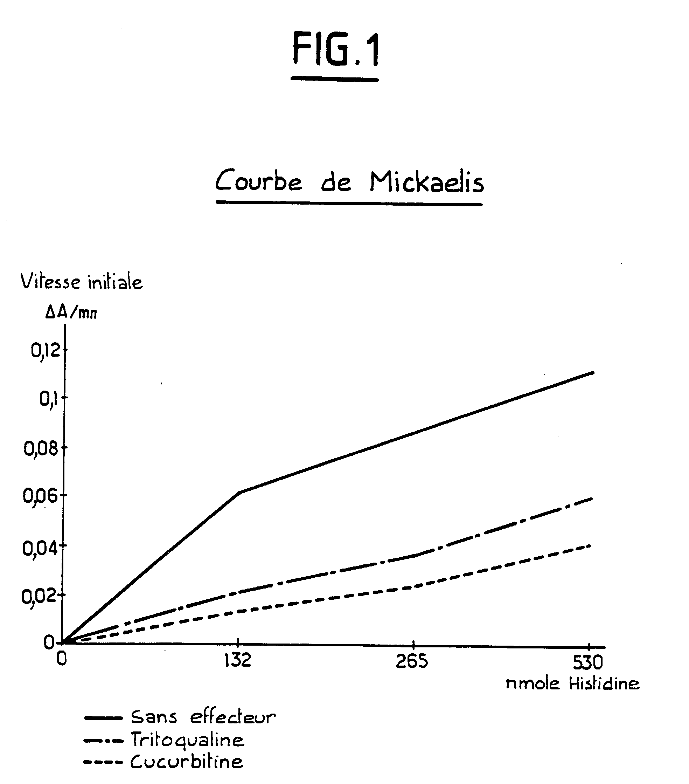

Les résultats obtenus avec diverses concentrations initiales d'histidine, à savoir à 132 nmol, 265 nmol, et 530 nmol, respectivement sans effecteur, avec tritoqualine comme inhibiteur d'HDC connu, et avec la cucurbitine sous forme racémique comme inhibiteur selon l'invention sont répertoriés au tableau II ci-après et font l'objet de la courbe de Mickaelis, objet de la figure 1.

**Tableau II**

| Histidine | 132 nmol | 265 nmol | 530 nmol |
|---|---|---|---|
| Sans effecteur | 0,062 | 0,089 | 0,1132 |
| Tritoqualine (art antérieur) | 0,021 | 0,0374 | 0,0604 |
| Cucurbitine (invention) | 0,013 | 0,0242 | 0,042 |

Il ressort clairement du tableau II et de la courbe de Mickaelis objet de la figure 1, que la cucurbitine est un inhibiteur d'HDC beaucoup plus puissant que la tritoqualine, ce qui constitue un résultat tout fait surprenant pour un homme de l'art.

### 1 - B par un dosage RIA

On peut également mettre en évidence l'activité anti-histaminique de la cucurbitine par un dosage radioimmunologique (Radio Immuno Assay ou "RIA") de la manière suivante.

Ce dosage a lieu en dosant l'histamine directement produite sous l'action de l'enzyme HDC, par la méthode RIA, bien connue à l'homme de l'art, et notamment décrite dans la notice d'utilisation d'un kit de dosage dénommé HISTAMINE Radioimmunoassay kit (Cat. # 1302) commercialisé par la Société IMMUNOTECH (Marseille-France).

On dose au cours du temps La quantité d'histamine libérée (en nanomoles) pour une concentration de 16.10⁻³ molaire d'histidine dans un tampon phosphate à pH 6,3, respectivement sans effecteur, avec la tritoqualine comme effecteur de comparaison et avec de la cucurbitine racémique de synthèse comme agent anti-histaminique selon l'invention. La tritoqualine et la cucurbitine sont utilisées à la concentration de 2.10⁻³ molaire.

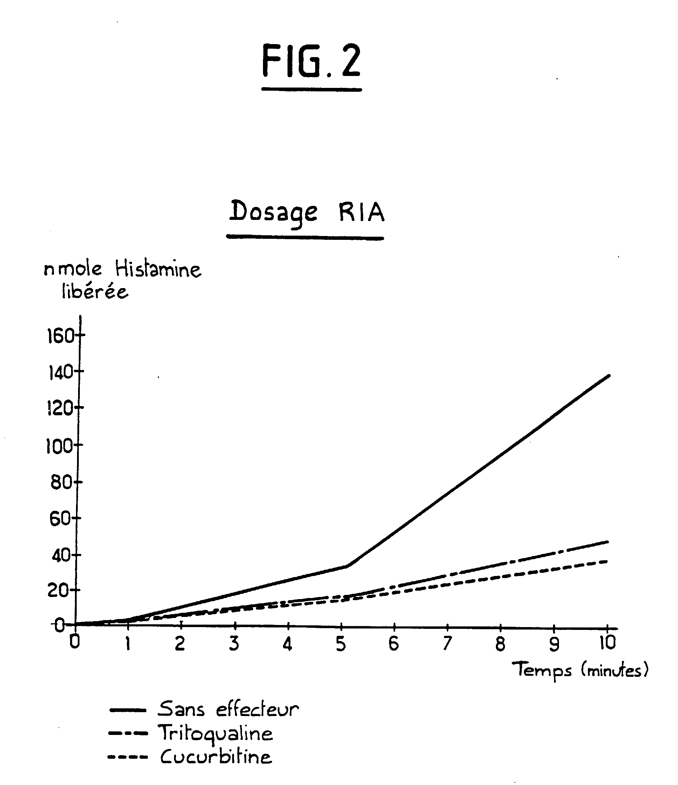

Les résultats obtenus exprimés en nanomoles d'histidine libérée sont répertoriés au tableau III ci-après et font l'objet de la courbe de la figure 2 où l'on a indiqué en ordonnées le nombre de nanomoles d'histamine libérée et en abscisses le temps exprimé en minute. La courbe sans effecteur est tracée en trait continu, la courbe obtenue avec la tritoqualine est tracée en trait mixte et la courbe obtenue avec la cucurbitine est tracée en pointillés.

**TABLEAU III**

| Temps | 5 min** | 10 min** | % I*(à 5 min) | % I*(à 10 min) |
|---|---|---|---|---|
| Sans effecteur | 32,66 | 140,79 | 0 | 0 |
| Tritoqualine | 16,48 | 49,27 | 49 | 65 |
| Cucurbitine | 14,85 | 38,29 | 55 | 73 |

| | | | | |
|---|---|---|---|---|
| * I = Inhibition | | | | |
| ** = nanomoles d'histamine libérée | | | | |

On voit clairement à partir du tableau III que la quantité d'histamine libérée est nettement la plus faible en présence de cucurbitine.

Les résultats obtenus par la méthode RIA confirment donc que la cucurbitine a une activité inhibitrice de la formation d'histamine nettement plus puissante que la tritoqualine au bout de quelques minutes.

Divers exemples de formulation de compositions cosmétiques ou pharmaceutiques, notamment dermatologiques selon l'invention sont les suivants :

### Exemple 8

### Comprimés

Par comprimé pour administration orale :
- (±) cucurbitine 100 mg
- amidon 38 mg
- lactose 75 mg
- talc 10 mg
- autres excipients pour comprimés (dont stéarate de magnésium) qsp 250 mg

Indications : traitement préventif et curatif des manifestations allergiques, notamment cutanées et respiratoires. Posologie : 1 à 10 comprimés par jour chez l'adulte. Diminuer les doses par 2 chez l'enfant jusqu'à 15 ans.

### Exemple 9

### Poudre pour aérosol

Pour 100 g dans un flacon pressurisé :
- (±)cucurbitine 3 g
- mannitol 1 g
- gaz pulseurs 96 g

Indications : traitement préventif et curatif de toutes manifestations allergiques respiratoires telles que asthme bronchique et bronchites asthmatiformes.

Posologie : administration intrabronchique, à raison de 4 à 6 aspirations par jour en moyenne.

### Exemple 10

### Emulsion adoucissante pour peaux sensibles

- extrait de pépins selon l'exemple 5 titrant 3 % en (-) cucurbitine 10 g
- émulsion cosmétique classique pour peaux sensibles (alcools gras, alcools gras polyoxyéthylénés huile minérale, palmitate d'isopropyle, glycérine, gélifiant, conservateurs, parfums, eau) qsp 100 g

### Exemple 11

### Fond de teint hypoallergénique

- extrait de pulpe de Cucurbita pepo renforcé en (-)-cucurbitine selon l'exemple 4, ayant une teneur de 1 % en (-)-cucurbitine 10 g
- composition classique pour fond de teint (esters gras, squalane, lécithine de soja, silicone volatile, propylène glycol, gomme xanthane, filtre solaire, pigments, conservateurs, parfum, eau) qsp 100 g

### Exemple 12

### Mascara anti-irritant

- (±)-cucurbitine 1,0 g
- bleu outre-mer 9,0 g
- alcool hexadécylique 7,4 g
- propylèneglycol 9,0 g
- acide stéarique 11,3 g
- monostéarate de glycérol 4,4 g
- triéthylamine 3,6 g
- conservateur 0,3 g
- eau qsp... 100 g

## Revendications

1. Utilisation de la cucurbitine ou de l'un de ses sels ou esters, ou d'un extrait de plante en contenant, à condition qu'un extrait de pulpe de fruits de Cucurbitacée contienne au moins 0,5 % en poids en cucurbitine, comme agent cosmétique.

2. Utilisation de la cucurbitine ou de l'un de ses sels ou esters, ou d'un extrait de plante en contenant, à condition qu'un extrait de pulpe de fruits de Cucurbitacée contienne au moins 0,5 % en poids en cucurbitine, pour la préparation d'une composition pharmaceutique, notamment dermatologique, à activité antiallergique.

3. Utilisation selon la revendication 1 ou 2, sous forme d'une composition, caractérisée en ce ladite composition est destinée à la prévention ou au traitement des manifestations allergiques, notamment au niveau des bronches, de la peau et de l'oeil.

4. Utilisation selon l'une des revendications 1 à 3, caractérisée en ce que la cucurbitine est présente sous forme de l'un de ses sels ou de ses esters cosmétiquement ou pharmaceutiquement acceptables, tel que le mono- ou le dibromohydrate ou le mono- ou le dichlorohydrate, l'ester méthylique ou éthylique.

5. Utilisation selon l'une des revendications 1 à 4, caractérisée en ce que l'extrait de plante précité est un extrait de Cucurbitacée, en particulier de Cucurbita maxima Duch, de Cucurbita pepo L. ou de Cucurbita moschata ; de préférence, il s'agit d'un extrait de pépins de Cucurbitacée.

6. Utilisation selon l'une des revendications 1 à 5, caractérisée en ce que l'extrait de plante précité est un extrait de pulpe de fruits de Cucurbitacée contenant au moins 0,5 % en poids de cucurbitine.

7. Utilisation selon l'une des revendications 1 à 6, sous forme d'une composition, caractérisée en ce que ladite composition contenant la cucurbitine ou un de ses sels ou esters, contient en outre des vésicules de type liposome, en particulier, la cucurbitine, son sel ou ester ou l'extrait de plante précité étant encapsulé au moins en partie dans lesdites vésicules de type liposome.

8. Utilisation selon l'une des revendications 1 à 7, caractérisée en ce que la cucurbitine ou l'un de ses sels ou esters cosmétiquement ou pharmaceutiquement acceptables est présente à une concentration de 0,001 % à 10 %, de préférence de 0,01 % à 5 %, en poids de la composition totale.

9. Composition cosmétique, caractérisée en ce qu'elle comprend, comme ingrédient actif, de la cucurbitine ou l'un de ses sels ou esters cosmétiquement acceptables, ou un extrait de plante en contenant, à la condition qu'un extrait de pulpe de fruits de Cucurbitacée contienne au moins 0,5 % en poids en cucurbitine, éventuellement dans un excipient, véhicule ou support cosmétiquement acceptable.

10. Composition cosmétique selon la revendication 9, caractérisée en ce que l'extrait de plante précité est un extrait de Cucurbitacée, en particulier de Cucurbita maxima Duch, de Cucurbita pepo L. ou de Cucurbita moschata ; de préférence, il s'agit d'un extrait de pépins de Cucurbitacée.

11. Composition cosmétique selon la revendication 9 ou 10, caractérisée en ce qu'il s'agit d'un extrait de pulpe de fruits de Cucurbitacée contenant au moins 0,5 % en poids en cucurbitine.

12. Composition cosmétique selon l'une des revendications 9 à 11, caractérisée en ce que la cucurbitine ou l'un de ses sels ou esters cosmétiquement acceptables est présente en une quantité efficace pour présenter une activité antiallergique, en particulier à une concentration de 0,001 % à 10 %, de préférence de 0,01 % à 5 %, en poids de la composition totale.

13. Composition pharmaceutique, notamment dermatologique, à activité antiallergique, caractérisée en ce qu'elle comprend comme ingrédient actif, de la cucurbitine ou l'un de ses sels ou esters pharmaceutiquement acceptables, ou un extrait de plante en contenant, à la condition qu'un extrait de pulpe de fruits de Cucurbitacée contienne au moins 0,5 % en poids en cucurbitine, en une quantité efficace pour présenter une activité antiallergique, notamment au niveau des bronches, de la peau et de l'oeil, éventuellement dans un excipient, véhicule ou support pharmaceutiquement acceptable.

14. Composition pharmaceutique selon la revendication 13, caractérisée en ce que l'extrait de plante précité est un extrait de Cucurbitacée, en particulier de Cucurbita maxima Duch, de Cucurbita pepo L; ou de Cucurbita moschata ; de préférence, il s'agit d'un extrait de pépins de Cucurbitacée.

15. Composition pharmaceutique selon la revendication 13 ou 14, caractérisée en ce que l'extrait de plante précité est un extrait de pulpe de fruits de Cucurbitacée contenant au moins 0,5 % en poids en cucurbitine.

16. Composition pharmaceutique selon l'une des revendications 13 à 15, caractérisée en ce qu'elle est destinée à l'usage topique et que la concentration en cucurbitine, ou en ses sels ou esters, est comprise entre 0,001 % et 10 %, de préférence entre 0,01 % et 5 %.

17. Composition selon l'une des revendications 9 à 16, caractérisée en ce qu'elle contient en outre des vésicules de type liposome, en particulier la cucurbitine, son sel ou ester ou l'extrait de plante précité étant encapsulé au moins en partie dans lesdites vésicules de type liposome.

18. Composition selon l'une des revendications 9 à 17, caractérisée en ce que la cucurbitine est présente sous forme lévogyre.

19. Composition selon la revendication 18, caractérisée en ce que la cucurbitine est obtenue à partir de pépins ou de pulpe de fruits de Cucurbitacée, en particulier de Cucurbita maxima Duch., de Cucurbita pepo L., ou de Cucurbita moschata Duch.

20. Procédé pour diminuer le potentiel allergisant d'une composition cosmétique ou pharmaceutique, notamment dermatologique, caractérisé en ce qu'on incorpore à ladite composition une quantité efficace de cucurbitine sous forme libre ou sous forme de l'un de ses sels ou esters cosmétiquement ou pharmaceutiquement acceptables, ou d'un extrait de plante en contenant, de sorte que la composition finale présente un risque allergisant réduit.

21. Procédé selon la revendication 20, caractérisé en ce que l'extrait de plante précité est un extrait de pulpe de fruits de Cucurbitacée contenant au moins 0,5 % en poids en cucurbitine.

22. Procédé selon la revendication 20 ou 21, caractérisée en ce que la concentration en cucurbitine, ou en ses sels ou esters, est comprise entre 0,001 % et 10 %, de préférence entre 0,01 % et 5 %.

23. Procédé selon l'une des revendications 20 à 22, caractérisé en ce que l'extrait de plante précité est un extrait de Cucurbitacée, en particulier de Cucurbita maxima Duch., de Cucurbita pepo L., ou de Cucurbita moschata Duch, de préférence il s'agit d'un extrait de pépins de Cucurbitacée.

24. Procédé selon l'une des revendications 20 à 23, caractérisé en ce qu'on incorpore en outre à ladite composition des vésicules de type liposome, en particulier la cucurbitine, son sel ou ester ou l'extrait de plante précité étant encapsulé au moins en partie dans lesdites vésicules de type liposome.

25. Procédé de synthèse de la cucurbitine, caractérisé en ce qu'on utilise comme produit de départ de la 1-benzyl-3-pyrrolidinone.

26. Procédé selon la revendication 25, caractérisé en ce qu'on traite la 1-benzyl-3-pyrrolidinone avec une solution ammoniacale de chlorure d'ammonium et de cyanure de potassium, en obtenant la (±) 1-benzyl-3-amino-3-cyanopyrrolidine que l'on transforme ensuite par hydrolyse acide ou basique en acide (±) 3-amino-1-benzyl-3-pyrrolidinecarboxylique, puis on procède à une réduction hydrogénée, de préférence une hydrogénolyse catalytique pour obtenir l'acide (±) 3-amino-3-pyrrolidine carboxylique ou (±) cucurbitine.

27. Procédé selon la revendication 25 ou 26, caractérisé en ce qu'on réalise la réaction de la 1-benzyl-3-pyrrolidinone avec le chlorure d'ammonium et le cyanure de potassium dans un rapport molaire 1/4/4 à la température ambiante pendant au moins 48 h.

28. Procédé selon l'une des revendications 25 à 27, caractérisé en ce qu'on isole l'isomère lévogyre de l'acide 3-amino-3-pyrrolidinecarboxylique à partir du mélange racémique, en particulier par l'intermédiaire de la préparation des diastéréoisomères.

## Claims

1. A use of cucurbitine or one of its salts or esters, or of a plant extract containing it, provided that an extract of Cucurbitaceae fruit pulp contains at least 0.5% by weight of cucurbitine, as cosmetic agent.

2. The use of cucurbitine or one of its salts or esters, or of a plant extract containing it, provided that an extract of Cucurbitaceae fruit pulp contains at least 0.5% by weight of cucurbitine, for the preparation of a pharmaceutical, in particular dermatological, composition having antiallergic activity.

3. The use according to Claim 1 or 2, in the form of a composition, characterised in that said composition is intended for the prevention or treatment of allergic manifestations, in particular in the bronchi, skin and eye.

4. The use according to one of Claims 1 to 3, characterised in that cucurbitine is present in the form of one of its cosmetically or pharmaceutically acceptable salts or esters, such as the mono- or dihydrobromide or the mono- or dihydrochloride or the methyl or ethyl ester.

5. The use according to one of Claims 1 to 4, characterised in that the abovementioned plant extract is an extract of Cucurbitaceae, especially of Cucurbita maxima Duch., of Cucurbita pepo L. or of Cucurbita moschata; preferably, it is an extract of Cucurbitaceae pips.

6. The use according to one of Claims 1 to 5, characterised in that the abovementioned plant extract is an extract of Cucurbitaceae fruit pulp containing at least 0.5% by weight of cucurbitine.

7. The use according to one of Claims 1 to 6, in the form of a composition, characterised in that said composition containing cucurbitine or one of its salts or esters contains, in addition, vesicles of the liposome type, the cucurbitine, its salt or ester or the abovementioned plant extract being, in particular, at least partially encapsulated in the said vesicles of the liposome type.

8. The use according to one of Claims 1 to 7, characterised in that cucurbitine or one of its cosmetically or pharmaceutically acceptable salts or esters is present at a concentration of 0.001% to 10%, and preferably 0.01% to 5% by weight of the total composition.

9. A cosmetic composition, characterised in that it comprises, as active ingredient cucurbitine or one of its cosmetically acceptable salts or esters, or a plant extract containing it, provided that an extract of Cucurbitaceae fruit pulp contains at least 0.5% by weight of cucurbitine, where appropriate in a cosmetically acceptable excipient, vehicle or carrier.

10. The cosmetic composition according to Claim 9, characterised in that the abovementioned plant extract is an extract of Cucurbitaceae, especially of Cucurbita maxima Duch., of Cucurbita pepo L. or of Cucurbita moschata; preferably it is an extract of Cucurbitaceae pips.

11. The cosmetic composition according to claim 9 or 10, characterised in that the extract in question is an extract of Cucurbitaceae fruit pulp containing at least 0.5% by weight of cucurbitine.

12. The cosmetic composition according to one of Claims 9 to 11, characterised in that cucurbitine or one of its cosmetically acceptable salts or esters is present in an amount which is effective for displaying antiallergic activity, especially at a concentration of 0.001% to 10%, and preferably 0.01% to 5%, by weight of the total composition.

13. A pharmaceutical, in particular dermatological, composition having antiallergic activity, characterised in that it comprises, as active ingredient, cucurbitine or one of its pharmaceutically acceptable salts or esters, or a plant extract containing it, provided that an extract of Cucurbitaceae fruit pulp contains at least 0.5% by weight of cucurbitine, in an amount which is effective for displaying antiallergic activity, in particular in the bronchi, skin and eye, where appropriate in a pharmaceutically acceptable excipient, vehicle or carrier.

14. The pharmaceutical composition according to Claim 13, characterised in that the abovementioned plant extract is an extract of Cucurbitaceae, especially of Cucurbita maxima Duch., Cucurbita pepo L. or Cucurbita moschata; preferably, it is an extract of Cucurbitaceae pips.

15. The pharmaceutical composition according to Claim 13 or 14, characterised in that the abovementioned plant extract is an extract of Cucurbitaceae fruit pulp containing at least 0.5% by weight of cucurbitine.

16. The pharmaceutical composition according to one of Claims 13 to 15, characterised in that it is intended for topical use and in that the concentration of cucurbitine or of its salts or esters is between 0.001% and 10% and preferably between 0.01% and 5%.

17. A composition according to one of Claims 9 to 16, characterised in that it contains, in addition, vesicles of the liposome type, the cucurbitine, its salt or ester or the abovementioned plant extract being, in particular, at least partially encapsulated in the said vesicles of the liposome type.

18. The composition according to one of Claims 9 to 17, characterised in that cucurbitine is present in laevorotatory form.

19. The composition according to Claim 18, characterised in that the cucurbitine is obtained from pips or fruit pulp of Cucurbitaceae, especially of Cucurbita maxima Duch., of Cucurbita pepo L. or of Cucurbita moschata Duch.

20. A process for decreasing the allergenic potential of a cosmetic or pharmaceutical, in particular dermatological, composition, characterised in that an effective amount of cucurbitine, in free form or in the form of one of its cosmetically or pharmaceutically acceptable salts or esters, or of a plant extract containing it, is incorporated in the said composition so that the final composition displays a reduced risk of being allergenic.

21. The process according to Claim 20, characterised in that the abovementioned plant extract is an extract of Cucurbitaceae fruit pulp containing at least 0.5% by weight of cucurbitine.

22. The process according to Claim 20 or 21, characterised in that the concentration of cucurbitine or of its salts or esters is between 0.001% and 10%, and preferably between 0.01% and 5%.

23. The process according to one of Claims 20 to 22, characterised in that the abovementioned plant extract is an extract of Cucurbitaceae, especially of Cucurbita maxima Duch., of Cucurbita pepo L. or of Cucurbita moschata Duch., preferably it is an extract of Cucurbitaceae pips.

24. The process according to one of Claims 20 to 23, characterised in that vesicles of the liposome type, the cucurbitine, its salt or ester or the abovementioned plant extract being, in particular, at least partially encapsulated in the said vesicles of the liposome type are incorporated, in addition, to the said composition .

25. A process for the synthesis of cucurbitine, characterised in that 1-benzyl-3-pyrrolidinone is used as starting product.

26. The process according to Claim 25, characterised in that 1-benzyl-3-pyrrolidinone is treated with an ammonical solution of ammonium chloride and of potassium cyanide, (±)-1-benzyl-3-amino-3-cyanopyrrolidine being obtained, which compound is then converted by acid or basic hydrolysis to (±)-3-amino-1-benzyl-3-pyrrolidinecarboxylic acid, and a reduction with hydrogen, preferably a catalytic hydrogenolysis, is thereafter carried out to obtain (±)-3-amino-3-pyrrolidinecarboxylic acid or (±)-cucurbitine.

27. The process according to Claim 25 or 26, characterised in that the reaction of 1-benzyl-3-pyrrolidinone with ammonium chloride and the potassium cyanide is carried out in a 1:4:4 mole ratio at room temperature for at least 48 h.

28. The process according to one of Claims 25 to 27, characterised in that the laevorotatory isomer of 3-amino-3-pyrrolidinecarboxylic acid is isolated from the racemic mixture, especially via the preparation of diastereoisomers.

## Patentansprüche

1. Verwendung von Cucurbitin oder eines seiner Salze oder Ester, oder eines dieses bzw. diesen enthaltenden Pflanzen-Extrakts, mit der Maßgabe, daß ein Fruchtfleisch-Extrakt von Cucurbitaceae zumindest 0,5 Masse-% Cucurbitin enthält, als kosmetisches Mittel.

2. Verwendung von Cucurbitin oder eines seiner Salze oder Ester, oder eines dieses bzw. diesen enthaltenden Pflanzen-Extrakts, mit der Maßgabe, daß ein Fruchtfleisch-Extrakt von Cucurbitaceae zumindest 0,5 Masse-% Cucurbitin enthält, bei der Herstellung einer pharmazeutischen, insbesondere dermatologischen, Zusammensetzung mit anti-allergischer Wirksamkeit.

3. Verwendung nach Anspruch 1 oder 2, in Form einer Zusammensetzung, dadurch gekennzeichnet, daß diese Zusammensetzung zur Prävention oder zur Behandlung allergischer Manifestationen, insbesondere auf der Ebene der Bronchien, der Haut und des Auges bestimmt ist.

4. Verwendung nach einem der Ansprüche 1 bis 3, dadurch gekennzeichnet, daß das Cucurbitin in Form eines seiner kosmetisch oder pharmazeutisch annehmbaren Salze oder Ester, wie Mono- oder Dibromhydrat oder Mono- oder Dichlorhydrat, Methyl- oder Ethylester, vorliegt.

5. Verwendung nach einem der Ansprüche 1 bis 4, dadurch gekennzeichnet, daß der Pflanzen-Extrakt ein Extrakt von Cucurbitaceae ist, insbesondere Cucurbita maxima Duch, Cucurbita pepo L. oder Cucurbita moschata, wobei es sich vorzugsweise um einen Kern-Extrakt von Cucurbitaceae handelt.

6. Verwendung nach einem der Ansprüche 1 bis 5, dadurch gekennzeichnet, daß der Pflanzen-Extrakt ein Fruchtfleisch-Extrakt von Cucurbitaceae ist, der zumindest 0,5 Masse-% Cucurbitin enthält.

7. Verwendung nach einem der Ansprüche 1 bis 6, in Form einer Zusammensetzung, dadurch gekennzeichnet, daß diese Zusammensetzung, die Cucurbitin oder eines seiner Salze oder einen seiner Ester enthält, außerdem Vesikel vom Liposomen-Typ enthält, wobei insbesondere das Cucurbitin, sein Salz oder Ester oder der Pflanzen-Extrakt zumindest teilweise in diesen Vesikeln vom Liposomen-Typ eingekapselt ist.

8. Verwendung nach einem der Ansprüche 1 bis 7, dadurch gekennzeichnet, daß das Cucurbitin oder eines seiner Salze oder einer seiner Ester, die kosmetisch oder pharmazeutisch annehmbar sind, bei einer Konzentration von 0,001 % bis 10 %, vorzugsweise 0,01 % bis 5 %, bezogen auf die Masse der gesamten Zusammensetzung, vorliegt.

9. Kosmetische Zusammensetzung, dadurch gekennzeichnet, daß sie als aktiven Bestandteil Cucurbitin oder eines seiner Salze oder einen seiner Ester, die kosmetisch annehmbar sind, oder einen dieses bzw. diesen enthaltenden Pflanzen-Extrakt umfaßt, mit der Maßgabe, daß ein Fruchtfleisch-Extrakt von Cucurbitaceae zumindest 0,5 Masse-% Cucurbitin, gegebenenfalls in einem kosmetisch annehmbaren Exzipienten, Vehikel oder Träger, enthält.

10. Kosmetische Zusammensetzung nach Anspruch 9, dadurch gekennzeichnet, daß der Pflanzen-Extrakt ein Extrakt von Cucurbitaceae ist, insbesondere Cucurbita maxima Duch, Cucurbita pepo L. oder Cucurbita moschata, wobei es sich vorzugsweise um einen Kern-Extrakt von Cucurbitaceae handelt.

11. Kosmetische Zusammensetzung nach Anspruch 9 oder 10, dadurch gekennzeichnet, daß es sich um einen Fruchtfleisch-Extrakt von Cucurbitaceae handelt, der zumindest 0,5 Masse-% Cucurbitin enthält.

12. Kosmetische Zusammensetzung nach einem der Ansprüche 9 bis 11, dadurch gekennzeichnet, daß das Cucurbitin oder eines seiner Salze oder einer seiner Ester, die kosmetisch annehmbar sind, in einer wirksamen Menge, um eine anti-allergische Wirksamkeit zu zeigen, insbesondere bei einer Konzentration von 0,001 % bis 10 %, vorzugsweise 0,01 % bis 5 %, bezogen auf die Masse der gesamten Zusammensetzung, vorliegt.

13. Pharmazeutische, insbesondere dermatologische, Zusammensetzung mit anti-allergischer Wirksamkeit, dadurch gekennzeichnet, daß sie als aktiven Bestandteil Cucurbitin oder eines seiner Salze oder einen seiner Ester, die pharmazeutisch annehmbar sind, oder einen dieses bzw. diesen enthaltenden Pflanzen-Extrakt, mit der Maßgabe, daß ein Fruchtfleisch-Extrakt von Cucurbitaceae zumindest 0,5 Masse-% Cucurbitin enthält, in einer wirksamen Menge, um eine anti-allergische Wirksamkeit zu zeigen, insbesondere auf der Ebene der Bronchien, der Haut und des Auges, gegebenenfalls in einem pharmazeutisch annehmbaren Exzipienten, Vehikel oder Träger, umfaßt.

14. Pharmazeutische Zusammensetzung nach Anspruch 13, dadurch gekennzeichnet, daß der Pflanzen-Extrakt ein Extrakt von Cucurbitaceae ist, insbesondere Cucurbita maxima Duch, Cucurbita pepo L. oder Cucurbita moschata, wobei es sich vorzugsweise um einen Kern-Extrakt von Cucurbitaceae handelt.

15. Pharmazeutische Zusammensetzung nach Anspruch 13 oder 14, dadurch gekennzeichnet, daß der Pflanzen-Extrakt ein Fruchtfleisch-Extrakt von Cucurbitaceae ist, der zumindest 0,5 Masse-% Cucurbitin enthält.

16. Pharmazeutische Zusammensetzung nach einem der Ansprüche 13 bis 15, dadurch gekennzeichnet, daß sie zur topischen Verwendung bestimmt ist, und daß die Konzentration von Cucurbitin, oder eines seiner Salze oder Ester, zwischen 0,001 % und 10 %, vorzugsweise zwischen 0,01 % und 0,5 %, beträgt.

17. Zusammensetzung nach einem der Ansprüche 9 bis 16, dadurch gekennzeichnet, daß sie außerdem Vesikel vom Liposomen-Typ enthält, wobei insbesondere das Cucurbitin, sein Salz oder Ester oder der Pflanzen-Extrakt zumindest teilweise in diesen Vesikeln vom Liposomen-Typ eingekapselt ist.

18. Zusammensetzung nach einem der Ansprüche 9 bis 17, dadurch gekennzeichnet, daß Cucurbitin in linksdrehender Form vorliegt.

19. Zusammensetzung nach Anspruch 18, dadurch gekennzeichnet, daß das Cucurbitin aus Kernen oder Fruchtfleisch von Cucurbitaceae, insbesondere Cucurbita Duch, Cucurbita pepo L. oder Cucurbita moschata Duch, erhalten wird.

20. Verfahren zur Verringerung des Allergiepotentials einer kosmetischen oder pharmazeutischen, insbesondere dermatologischen, Zusammensetzung, dadurch gekennzeichnet, daß in diese Zusammensetzung eine wirksame Menge an Cucurbitin in freier Form oder in Form eines seiner kosmetisch oder pharmazeutisch annehmbaren Salze oder Ester oder eines dieses bzw. diesen enthaltenden Pflanzen-Extrakts derart eingeschlossen wird, daß die End-Zusammensetzung ein reduziertes Allergierisiko aufweist.

21. Verfahren nach Anspruch 20, dadurch gekennzeichnet, daß der Pflanzen-Extrakt ein Fruchtfleisch-Extrakt von Cucurbitaceae ist, der zumindest 0,5 Masse-% Cucurbitin enthält.

22. Verfahren nach Anspruch 20 oder 21, dadurch gekennzeichnet, daß die Konzentration von Cucurbitin, oder eines seiner Salze oder Ester, zwischen 0,001 % und 10 %, vorzugsweise zwischen 0,01 % und 5 %, beträgt.

23. Verfahren nach einem der Ansprüche 20 bis 22, dadurch gekennzeichnet, daß der Pflanzen-Extrakt ein Extrakt von Cucurbitaceae ist, insbesondere Cucurbita Duch, Cucurbita pepo L. oder Cucurbita moschata Duch, wobei es sich vorzugsweise um einen Kern-Extrakt von Cucurbitaceae handelt.

24. Verfahren nach einem der Ansprüche 20 bis 23, dadurch gekennzeichnet, daß außerdem in die Zusammensetzung Vesikel vom Liposomen-Typ eingeschlossen werden, wobei insbesondere das Cucurbitin, sein Salz oder Ester oder der Pflanzen-Extrakt zumindest teilweise in diese Vesikel vom Liposomen-Typ eingekapselt werden.

25. Verfahren zur Synthese von Cucurbitin, dadurch gekennzeichnet, daß als Ausgangsprodukt 1-Benzyl-3-pyrrolidinon verwendet wird.

26. Verfahren nach Anspruch 25, dadurch gekennzeichnet, daß 1-Benzyl-3-pyrrolidinon mit einer ammoniakalischen Lösung von Ammoniumchlorid und Kaliumcyanid behandelt wird, wobei (±)-1-Benzyl-3-amino-3-cyanopyrrolidin erhalten wird, das anschließend durch Säure- oder Basenhydrolyse zu (±)-3-Amino-1-benzyl-3-pyrrolidincarbonsäure transformiert wird, dann eine hydrierte Reduktion, vorzugsweise eine katalytische Hydrogenolyse, durchgeführt wird, wobei (±)-3-Amino-3-pyrrolidincarbonsäure oder (±)-Cucurbitin erhalten wird.

27. Verfahren nach Anspruch 25 oder 26, dadurch gekennzeichnet, daß das Umsetzen von l-Benzyl-3-pyrrolidinon mit Ammoniumchlorid und Kaliumcyanid in einem Molverhältnis von 1/4/4 bei Umgebungstemperatur während zumindest 48 Stunden durchgeführt wird.

28. Verfahren nach einem der Ansprüche 25 bis 27, dadurch gekennzeichnet, daß das linksdrehende Isomer von 3-Amino-3-pyrrolidincarbonsäure aus der racemischen Mischung, insbesondere über den Zwischenschritt der Herstellung von Diastereomeren, isoliert wird.
